# EUROPEAN PATENT APPLICATION

(11) **EP 4 378 395 A1**
(43) Date of publication of application: **05.06.2024**
(21) Application number: 23020535.3
(22) Date of filing: 01.12.2023
(51) Int. Cl.: A61B 10/02

(54) **STYLET GUIDE-ASSISTED TRANSCUTANEOUS NEEDLE BIOPSY SYSTEM**

(30) Priority: 02.12.2022 GR 20220100998
(71) Applicant: Petsas, Theodoros, 22444 Larissa (GR)
(72) Inventor: Petsas, Theodoros, 22444 Larissa (GR)
(74) Representative: Malamis, Alkisti-Irene

(57) **Abstract**

An innovative biopsy needle system used in biopsies to obtain abnormal tissue from various organs for pathological examination is described. The difference with the needles currently used is that in the approach of the lesion a thin metal guide stylet is used, which approaches the lesion more easily with lower or even without complications. The puncture needle, of our proposal, consists of an outer cannula and an inner stylet. In our proposal the piercing needle shows a modification, its inner collar in its central position has a lumen along its entire length compatible in diameter with the diameter of the guide stylet. Thus, after the easy approach to the lesion by the guide stylet, the outer puncture needle, which carries the lumen inside with the stylet of the penetration needle may be forced forward and guided into the lesion. Then, by simultaneously removing the guide stylet with the stylet of the penetration needle, the sting of the piercing needle is released, forming a tunnel between the lesion and the outer space, through which a suitable pistol-type biopsy needle (GUN) passes to obtain the tissue biopsy.

## Description

### TECHNICAL FIELD OF THE INVENTION

The present invention relates to biopsy needles used in percutaneous biopsies and specifically to the use of a modified piercing needle, which with the use of a guide stylet assists the direction of the needle towards the lesion.

### PRIOR ART AND EVALUATION THEREOF

### Related Prior Knowledge - Brief History

Biopsy needles are used to obtain material from lesions, mainly malignant tumors, in various organs of the body. The lesions are found with imaging tests, e.g. Ultrasound (U/S), Computed Tomography (C-T) and in some cases with Magnetic Tomography (MRI). In percutaneous biopsies, the needle is inserted through the skin and guided either by US, CT or MRI. The technique of obtaining histological material consists of its percutaneous acquisition by gradually inserting a needle into the skin, then into the body wall (e.g. chest wall, abdominal wall) and then into the organ under examination (such as lung, liver, kidney, etc.) where the fault is located. Then the biopsy needle takes tissue samples from the lesion under examination, so that this material can be examined either in the cytological or in the pathology laboratory, in order to establish the final diagnosis. The other receiving methods e.g. the laparoscopic or the others, are technically more difficult and with more complications.

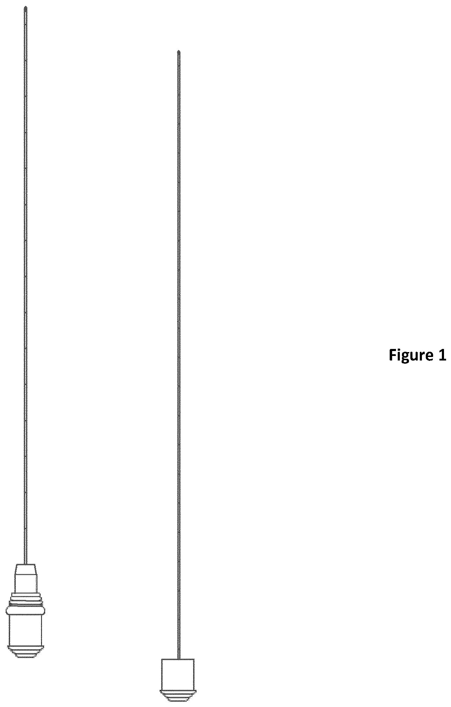

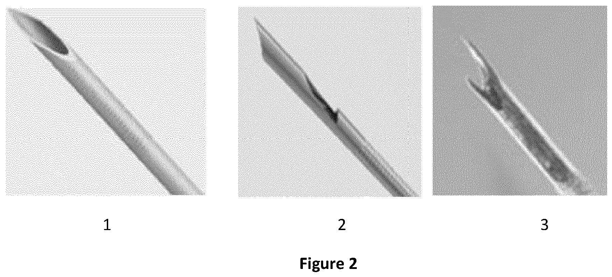

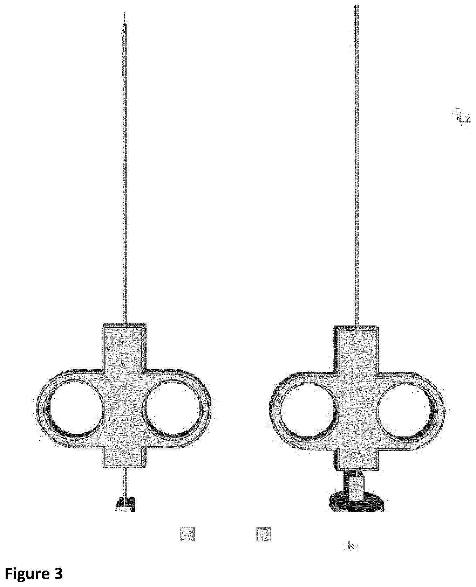

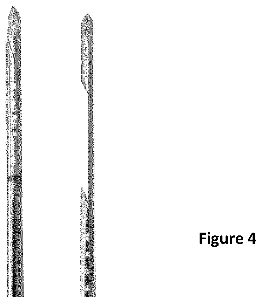

The percutaneous biopsy method was first applied in the 1960s where, Dahlgrens and Nordstrom in 1967, used a very fine diameter Chiba type penetration needle (very small gauge needle) (Figure 1) and approached the lesion under fluoroscopic guidance. It should be noted that in addition to the very fine-diameter needle for obtaining material from the lesion, a large number of fine-type biopsy needles with a modified tip were also manufactured to obtain a larger amount of material from the lesion. (Figure 2). In 1967, at the same time, in place of the simple biopsy needle, more complex needles began to be used, the most prevalent of which is the pistol-type needle (Figure 3) which, with the use of a spring inside it and a special configuration at its end (Figure 4), achieves reception older ones and more intact tissue sections, Ragde H. and Aldade H.C. in 1967. More precise needle placement was achieved using a CT scanner in the 70s by Haaga J.R. and Reich N.E. in 1977; it should be mentioned that the quantity and quality of the biopsy material, which are obtained with the pistol needles, are decisive for the accurate diagnosis of the disease. Today, when the patho-anatomical techniques have greatly improved (identification of tumor gene mutations, etc.), they are now modifying the therapeutic manipulations.

Biopsy techniques throughout this time to date have been modified in terms of the type of needles used, guidance techniques, improvement in the processing and interpretation of the obtained biopsy material as well as techniques to prevent and avoid and treat the complications of the method.

### Diathermal biopsy technique and method evaluation

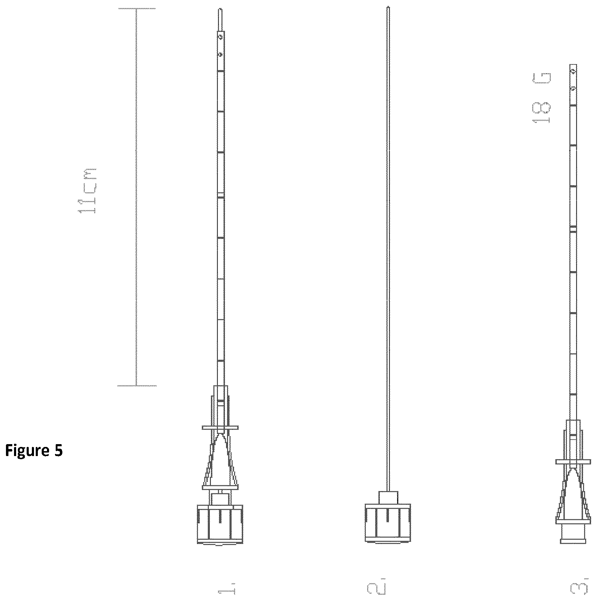

A biopsy with a pistol needle (gun type) is done as follows: Histological material is obtained using a coaxial needle system, which includes: the placement of a needle called penetration needle (Figure 5.1), which consists externally of a cannula (Figure 5.3) that bears lines on the surface of the one centimeter to aid in estimating its depth within the patient's body and internally by a cannula (Figure 5.2), which reinforces the cannula and with its anterior distal (terminal) end which is sharply pointed aids the insertion of the puncture needle within the tissues. After placing the puncture needle into the lesion, the puncture needle barb is removed, thus freeing the cannula lumen. Through the cannula, after removing the stylet of the puncture needle, it is possible to place, through it, a gun-type needle (Figure 3) with its specially shaped tip (Figure 4), and then the gun-type needle is fired and the material for examination.

It must be emphasized, however, that this method, as currently applied, presents three important technical weaknesses:
I. The difficulty of approaching the location of the damage in a moderate, difficult to very difficult degree, such as the small and deep lesions in the organs under biopsy.
II. The need to reposition the puncture needle, in cases where it has not taken the correct direction towards the damage, in which case the need to reposition it with one or more attempts arises, with the result that the repositioning cause injury to the vessels of the capsule and the parenchyma of the punctured organs as well as of these intraparenchymal vessels and especially in the lungs where the pleura is eaten.
III. The occurrence of complications such as bleeding in the punctured organs and the development of bleeding and pneumothorax in lung punctures. It should be mentioned that in terms of complications, these are basically two.

Bleeding that is a function of the thickness of the needle (GAUGE: Unit of measurement of the diameter of the needle, in **Table 1** where the ratio of the diameter of the very thin needle in millimetres and inches is shown) because the larger its diameter, the more it injures the vessels of the organ being punctured, resulting in greater bleeding.

The larger the diameter of the needle, the stiffer it is and the more shear it can make in the capsule of the punctured organ and the pleura (the pleura is the mucous membrane that surrounds the lung). If this breaks, then there is an exit of the air present in the lungs towards the chest cavity, a collapse of the corresponding lung and its non-participation in the respiratory function. The presence of air in the chest cavity is called a pneumothorax and is a very serious complication that can even cause death.

### ADVANTAGES OF THE PRESENT INVENTION

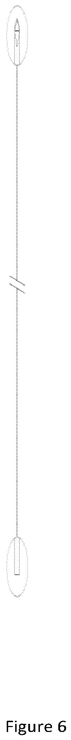

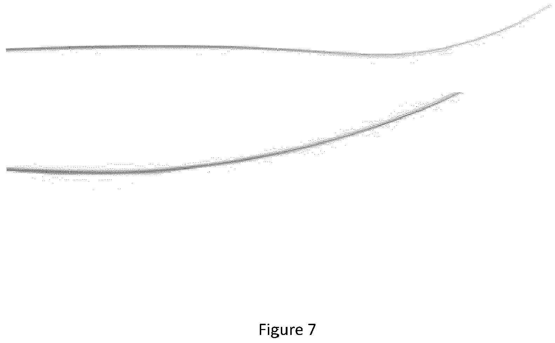

The purpose of the present invention is to provide a solution to the above problems and in particular to achieve the easiest approach to lesions for biopsy, while simultaneously reducing the complications presented by the method as applied to date. This is achieved by the following system:
A. Selection and use of a thin diameter and therefore flexible guide stylet (Figure 6) which causes much less injury to the vessels and therefore less bleeding. Because of its diameter it causes a very small pleural fissure and therefore a smaller pneumothorax. Another advantage of the guide stylet of the present invention is that during its stay within the parenchymal organs, and to a greater extent within the lung, during the operation, it has the property and bends more easily following the respiratory movements, thus reducing the complications.
B. The wire guide of the present invention can be repositioned more than once towards the lesion and if it does not approach it with the initial attempt, this can be repeated, causing far fewer complications than if this were done with the much larger-gauge puncture needle repeatedly.
C. Because the front end of the wire guide (guide stylet) can be bent, (Figure 7), it allows us to modify its path to the lesion and by changing it to be guided into it.

### DESCRIPTION OF THE INVENTION

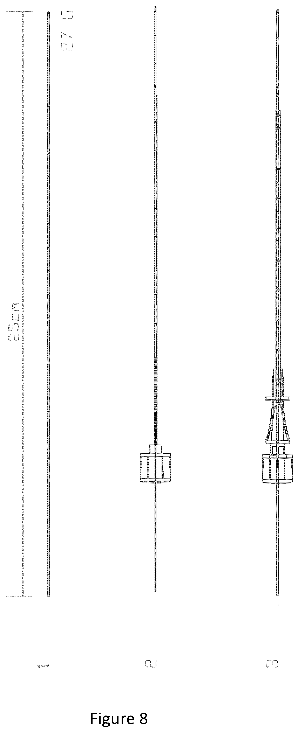

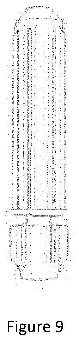

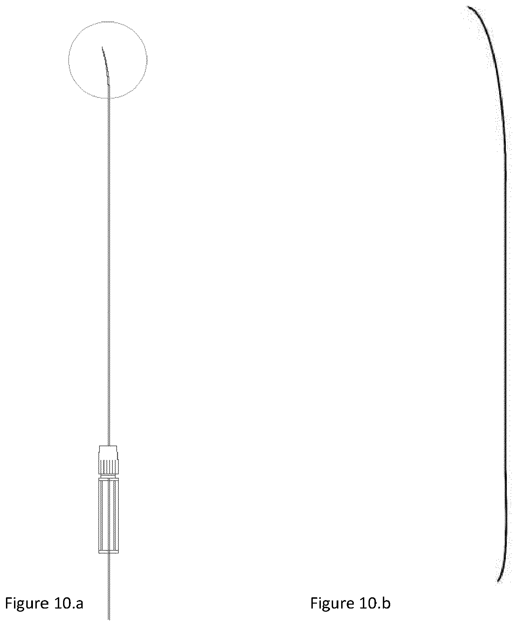

The proposed invention is a system which includes:
1. A thin diameter guide stylet (shown in Figure 6)
2. A modified penetration needle system (illustrated in Figure 8)
3. One very fine gauge (Chiba type) needle (shown in Figure 1)
4. A gun-type needle (shown in Figure 3) and
5. A torquer (illustrated in Figures 9 and 10a).

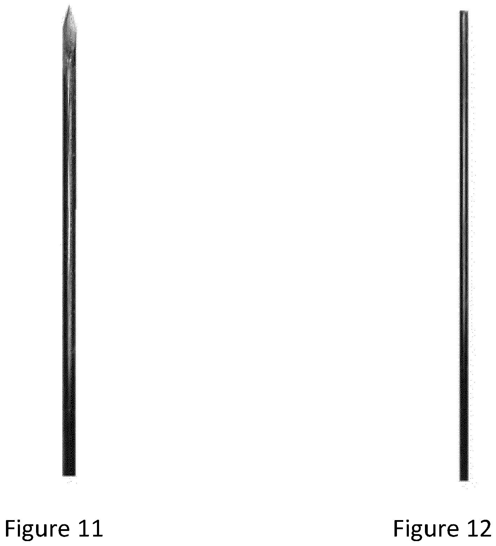

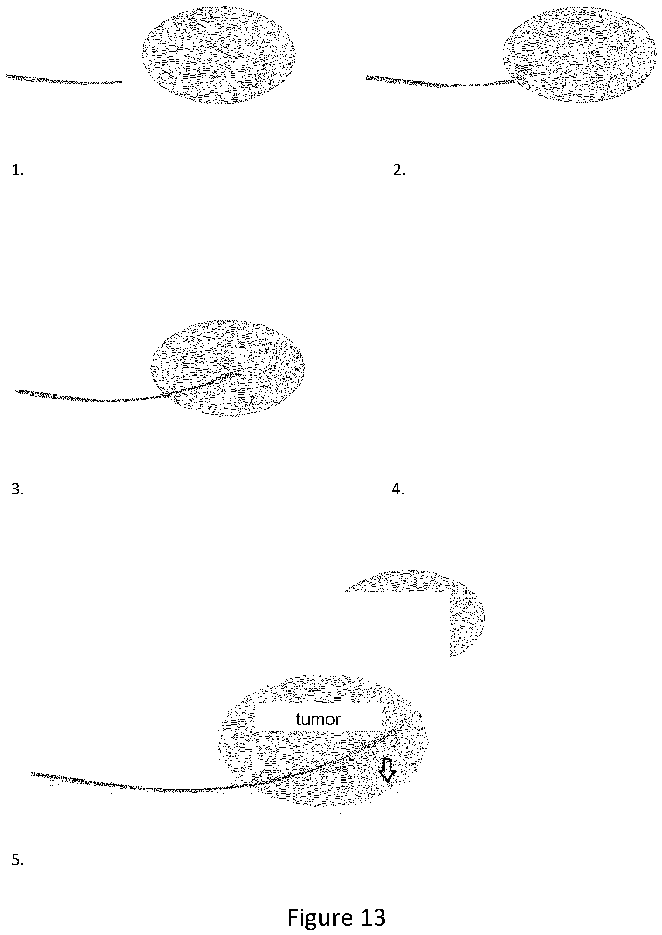

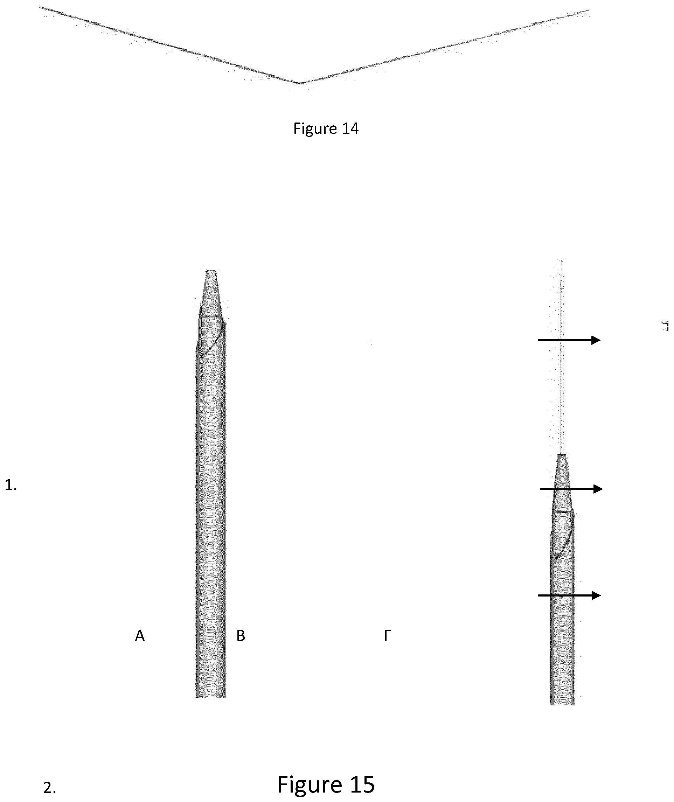

**1. The guide stylet: Technical characteristics.** It is a small gauge, metallic wire guide (shown in Figure 6) preferably 18 to 27 gauge thick, (dimensions of which are in inches or mm shown in **Table 1)** and typically 15-30 cm long. The guide stylet can be of different length and diameter, depending on the depth of the lesion, the length and thickness of the puncture needle as well as the length of the pistol biopsy needle to be used. The guide stylet is preferably of alloy steel so that it penetrates the tissue layers on its way to the lesion and does not bend when the puncture needle is slid over it and penetrates hard tissues. At the same time, it can be bent with appropriate handling (Figure 7). This is determined by the quality of the steel from which it is made, indicatively **Table 2).** The alloy of the steel must be such that it can penetrate the tissues, but also be able to be bent into the desired shape (Figure 10.b), which is maintained after exiting the very fine diameter needle or its lumen of the puncture needle. Also the guide stylet must be flexible at the same time, so that it can return to its shape when bent, so that when it comes out of the pin, which straightens it while it is inside its lumen, withdrawing it maintains the bend that formed before its insertion into the lumen of the penetration needle stylet. The steel characteristics should be as shown in Table 2, namely: bending strength at maximum load to be 2072 plus/minus 20Mpa, bending strength at breaking point to be 2062 plus/minus 10Mpa, bending strength at yield point to be 1796 plus/minus 30Mpa, breaking at elongation (pull) % to be 3 plus/minus 40, maximum elasticity to be 116 plus/minus 20Gpa.
   Also, the anterior distal (terminal) end of the guide stylet must be sharp in shape with various configurations, preferably diamond-like (as shown in Figures 6 and 11) for easier penetration and also must be echo-reflective for detection, when the penetration is performed with ultrasound guidance. The posterior distal end of the guide stylet should preferably be flat (as illustrated in Figures 6 and 11) for cases where the needle stylet placed with its leading edge being sharp does not stabilize within and penetrate the lesion. In these cases, the guide stylet is withdrawn and reversed which due to its shape (flat), has no penetration and is stabilized within it, and then the biopsy process continues. The guide stylet must additionally have the ability to bend as desired at its anterior distal end (Figure 7) to change its direction when appropriate when approaching the lesion. Also, preferably the guide stylet needle should have markings on its surface every one centimeter similar to the cannula of the puncture needle, which helps in estimating its depth within the patient's body. The use of the guide stylet is to aim, guide, as well as change the direction of its course towards the lesion and subsequently the correct direction of the puncture needle towards the lesion.
**2. The modified penetration needle. (****Figure 8****):** The penetration needle of the biopsy system proposed by the invention differs from the corresponding puncture needles known to date. In the present invention, the puncture needle stylet has along and within it a borehole of a caliber commensurate with the caliber of the puncture needle stylet (Figure 8.1, Figure 8.2, Figure 8.3 and Figure 14) so that its diameter allows the passage of the guide stylet along the entire length of the puncture needle stylet (Figure 8.3). That is, the column of the penetration needle has along its length and inside a suitable bore, which fits exactly, so that its diameter allows the passage through the guide stylet (Figure 8.2). It must be emphasized that there must be a perfect fit between the guide stylet and the bore stylet of the puncture needle because: A) When the penetration needle is inserted into the tissues and there is a gap between its bore stylet and the guide stylet the interstitial tissues it penetrates can enter the gap and implant into the lesion. This fact can alter the diagnosis of the pathology of the lesion. B) In cases where the puncture needle is passed through hard tissue, if the guide stylet and the bore stylet are not in perfect fit, the guide stylet may be deformed into existing bending and permanent deformation (cracking) and thus the attempt to fail (Figure 14). C) If there is no gap between the guide stylet and the bore of the puncture needle, it slides more easily into the tissues it pierces. Figure 15 illustrates the configuration of the front end (nose) of the modified penetration needle, which is also the object of the present invention.
**3. A torquer:** The system includes a torquer (Figure 9, 10.a), which locks the guide stylet along its axis. The torquer has an alignment along its longitudinal axis, which enables when the longitudinal alignment of the torquer is paralleled with the forward bend of the guide stylet, which is done as described below, the direction of the guide stylet bend within the of the patient's body, from the back of the guide stylet, which is outside the body and is visible. Thus, due to the alignment of the torquer of the invention, there may be a perception of the bending of the front curved end of the guide stylet, which is perceived by the alignment of the torquer. Therefore when it is perceived that the direction of the guide stylet deviates from the lesion, by withdrawing it and rotating it appropriately after reinsertion of the puncture needle stylet, with the help of the torquer, a change of direction can be achieved. With this appropriate manipulation, the user can achieve the change of the direction of the guide stylet towards the targeted lesion. This operation is called step by step (Figure 13).
   It should be noted that the direction of the leading edge of the guide stylet can be sensed (replacing the torquer) by bending the trailing edge of the guide stylet to the same plane as its front bend (Figure 10.b). This can also be done using a fine-bore 5ml or 10ml syringe or similar curved fitting and the thumb to shape the posterior bend of the guide stylet as well as the anterior bend. Care must be taken to ensure that the anterior bend is at the same level as the posterior bend. This can also be prefabricated by the factory that will manufacture the entire SET of the proposed biopsy needle system (Figure 10b). Backward bending of the guide stylet will not affect the entire biopsy procedure because when the guide stylet is inside either the very fine diameter needle or the bore of the puncture needle stylet, it is straightened.
**4. A very fine gauge needle (CHIBA needle).** In addition, the whole system includes a thin CHIBA needle in diameter (Fig. 1) approximately up to 10 cm long. (The length of which may vary depending on the intended use) as an auxiliary, with a tube that has a similar diameter to the steering guide stylet and indeed larger so that the guide stylet can pass through it. After its placement (in the abdominal or chest wall) in the direction of the lesion, without entering the parenchyma of the organ, it enables the guide wire to be advanced directly to the lesion.

**Manipulation technique.** When the CHIBA needle does not go in the right direction, it is removed and re-inserted only into the abdominal or thoracic wall, and after the direction the chiba is reinserted and go toward the lesion, the effort is repeated. Also, the Chiba needle is used as a conduit when the guide stylet needs to have its front end bent appropriately to change its direction (step-by-step operation). The Chiba needle is then inserted into the parenchyma of the instrument and by partially advancing it when it needs partial straightening, it assists it in its final entry into the lesion (step by step manipulation) (Figure 13). At the end of the lesion approach procedure and after the guide stylet is inserted into it, the Chiba needle is withdrawn and the penetration needle is advanced onto the needle guide which is thus driven into the lesion (Figure 13.4 and 13.5). The stylet of the penetration needle and the guide stylet are then removed leaving only the puncture needle cannula within the lesion, allowing the GUN biopsy needle to enter through its lumen to receive material, one or more times.

In conclusion, our proposal is a modification of the aforementioned components of the already existing coaxial needle system, including the steering guide stylet and the coaxial style of the puncture needle, with the lumen inside. The stylet of the penetration needle which is also the proposed innovation, to help orientate the penetration needle in the correct path forward to the lesion to obtain the pathological tissue with a Gun biopsy pistol, and especially in a way that avoids bleeding and pneumothorax.

The advantages of the system presented for the first time by the present invention are:
i. Facilitates the technique of better approach to the target lesion, due to the use of the thin steering guide stylet.
ii. Obtaining material from difficult-to-approach lesions (e.g. small or difficult locations, such as centrally located mass lesions within the organ in question).
iii. Less complications because the guide stylet driver, due to its small caliber, typically 25G versus a 17G or 19G puncture needle **(Table 1)** when used as the initial insertion needle **(Table 1),** causes less injury to the punctured organ when entering and therefore fewer complications, during its penetration route. Due to the small caliber of the guide stylet is less-traumatic and so more than one attempt can be made to enter and approach the lesion, in case it is not successfully placed with the first or other attempts. Whereas with the previously known technique applied today this would have to be done with a 17G or 19G puncture needle, as mentioned above, that is, with a much larger caliber needle.

The system of the present invention retains all the advantages of the pre-existing established co-axial technique and introduces a new complementary element, which facilitates the approach of the target lesion in question, augmenting the successful tissue sampling of small and difficult to approach lesions, while reducing its complications.

The proposed system is advantageous over the previous technique because the guide stylet serves as a guide for the insertion of the penetration needle, with its internal co-axial stylet having a lumen (Figure 13.4 and 13.5), which driven by the guide stylet may enter the lesion from where the biopsy material is to be taken. Then, by removing the guide stylet, at the same time with the stylet of the penetration needle, the cannula remains inside the lesion, creating a communication tunnel or channel between the lesion and the rear hub of the penetration needle cannula through which the pistol biopsy needle (Gun) may be inserted and take one or more tissue samples. Given that the cannula of the penetration needle has the same dimensions as the pistol biopsy needles (Guns) currently used, and is compatible with the already commercially available pistol biopsy needles for receiving pathological material, it gives a significant commercial advantage to the proposed invention.

Finally, the novelty of our proposal is that the stylet of the penetration needle has along its length a thin lumen compatible in diameter with the guide stylet. This allows the whole co-axial system to be advanced with fewer complications inside the targeted organ and to drive the penetration needle, due to the configuration of its stylet system, straight into the lesion. In addition, the steering needle guide stylet, when not entering directly into the lesion, it has the ability to bend its front end (Figure 13), and change the direction using the CHIBA needle in combination with the torquer or with the appropriate bending of the posterior portion of the guide stylet as described above, where this is deemed appropriate, and to guide the lesion and thereby ultimately the course of the puncture needle into the lesion. Finally, the sound-reflecting tip of the guide stylet helps to identify it within the tissues when the biopsy is done with ultrasound, while the delineation on its surface every 1 cm helps to estimate its depth inside the human body.

| **TABLE 1** | | |
|---|---|---|
| **Needle Diameter Correspondence Table** | | |
| G (Needle diameter) | Inches | Mm |
| 10 | 0.134 | 3.4 |
| 11 | 0.12 | 3.05 |
| 12 | 0.109 | 2.77 |
| 13 | 0.095 | 2.41 |
| 14 | 0.083 | 2.11 |
| **15** | **0.072** | **1.83** |
| **16** | **0.065** | **1.65** |
| **17** | **0.058** | **1.473** |
| **18** | **0.049** | **1.245** |
| **19** | **0.042** | **1.067** |
| **20** | **0.035** | **0.889** |
| **21** | **0.032** | **0.813** |
| **22** | **0.028** | **0.711** |
| **23** | **0.025** | **0.64** |
| **24** | **0.022** | **0.57** |
| **25** | **0.020** | **0.508** |
| **26** | **0.018** | **0.46** |
| **27** | **0.016** | **0.41** |
| **28** | **0.014** | **0.36** |
| 29 | 0.013 | 0.34 |
| 30 | 0.012 | 0.31 |

| **TABLE 2** | | | | |
|---|---|---|---|---|
| **Bending strength at maximum load (MPa)** | **Bending strength at breaking point (MPa)** | **Bending strength at yield point (MPa)** | **Fracture in extension (pull) %** | **Maximum elasticity (Gpa)** |
| 2072 ± 20 | 2062 ± 10 | 1796 ± 30 | 3 ± 40 | 116 ± 20 |

## Claims

1. A puncture needle assembly for use in biopsies of various organs, comprising a guide stylet, a torquer, a modified penetration needle system, a very fine diameter needle (CHIBA type) (Figure 1) and a gun-type needle, which assembly is **characterized by** that
- the guide stylet is a small gauge 18 Gauge to 27 Gauge metal wire, alloy steel, with a sharp and sound-reflecting anterior distal end and the ability to bend as desired at its anterior distal end,
- the penetration needle includes in its stylet a borehole along its entire length, which borehole is compatible in diameter, with a perfect fit with the diameter of the guide stylet, through which the thin metal guide stylet passes,
- the torquer should be aligned along its longitudinal axis.

2. A device according to claim 1 wherein the guide stylet is indicatively 15-30 cm long and the quality of steel from which it is made has the following characteristics:
bending strength at maximum load to be 2072 plus/minus 20 Mpa, bending strength at breaking point being 2062 plus/minus 10 Mpa, bending strength at yield point being 1796 plus/minus 30Mpa, breaking at elongation (pull) % being 3 plus/minus 40, the maximum elasticity being 116 plus/minus 20Gpa, and the guide rod has a diamond-shaped front distal end and a flat rear distal end.

3. A device according to each of claims 1 and 2 which, instead of the torquer, comprises a 5ml or 10ml syringe or a corresponding curved fitting.

4. A device according to any one of claims 1 to 3 wherein the CHIBA needle has a length that varies depending on the intended use and has a bore that is of a similar caliber to the guide stylet and larger so that the guide stylet can pass through it - letter

5. A device according to any one of claims 1 to 4 wherein the puncture needle has the same dimensions as currently used gun type needles, and is compatible with already commercially available gun type needles.
